# EUROPEAN PATENT APPLICATION

(11) **EP 1 647 290 A2**
(43) Date of publication of application: **19.04.2006**
(21) Application number: 05019653.4
(22) Date of filing: 09.09.2005
(51) Int. Cl.: A61M 1/10, A61M 1/36, A61M 25/10

(54) **Apparatus for performing an aortal valvular surgery intervention on a beating heart**

(30) Priority: 18.10.2004 IT MI20041977
(71) Applicant: N.G.C. Medical S.p.A., 22060 Novedrate CO (IT)
(72) Inventor: Pepino, Paolo, 80123 Napoli (IT); Pini, Patrizia, 20062 Cassano d'Adda (Milano) (IT); Cremascoli, Eugenio, 20145 Milano (IT)
(74) Representative: Cicogna, Franco

(57) **Abstract**

An apparatus for performing an aortal valvular surgery intervention on a beating heart comprises an outer mechanical circuit including a biopump and an infusion aortal cannula and being characterized in that it further comprises a left atrial cannula adapted to occlude the mitral valve so as to directly convey oxygened blood through a raising aorta by the outer mechanical circuit, thereby allowing to perform a cross aortotomy for exposing the aortic valve to operate thereon in an "open sky" manner.

## Description

### BACKGROUND OF THE INVENTION

The present invention relates to an apparatus for performing an aortal valvular surgery intervention on a beating heart.

Up to now an aortal valve surgery intervention on a beating heart could not be performed because of a technical impossibility, by the surgeon, of accessing the aortic valve without putting the patient under an extracorporeal circulation condition, i.e. by stopping both the pulsating activity of the heart and the gaseous exchange activity of the lungs.

Actually, the use of the extracorporeal circulation would be very dangerous for old patients, with associated pathologies (diabetes or renal diseases).

On the other hand, the beating heart technique, i.e. without using the extracorporeal circulation, has been recently successfully used, however limitatedly to the miocardic revascularization range.

Such a technique of method is highly advantageous for patients affected by a comparatively high risk during a like operation, performed in an extra-corporeal circulation condition.

### SUMMARY OF THE INVENTION

Accordingly, the aim of the present invention is to provide an apparatus adapted to perform a novel operating technique.

Within the scope of the above mentioned aim, a main object of the present invention is to provide such an apparatus allowing to extend the advantages of a beating heart operation also to interventions for replacing and repairing the aortic valve and ascending or raising aorta.

According to one aspect of the present invention, the above mentioned aim and objects, as well as yet other objects, which will become more apparent hereinafter, are achieved by an apparatus for performing an aortal valvular surgery intervention on a beating heart, comprising an outer mechanical circuit, including a biopump and an infusion aortic cannula and being characterized in that said apparatus further comprises a left atrial cannula, designed for occluding the mitral valve, thereby directly conveying oxygeried blood to the ascending aorta through said outer mechanical circuit, so as to allow to perform a cross aortotomy, to expose the aortic valve to allow to operate thereon in an open sky condition.

### BRIEF DESCRIPTION OF THE DRAWINGS

Further characteristics and advantages of the present invention will become more apparent hereinafter from the following detailed disclosure of a preferred, though not exclusive, embodiment of the invention, which is illustrated, by way of an indicative, but not limitative, example, in the accompanying drawings, where:
Figure 1 is a schematic perspective view of a human earth showing the physiologic circulatory activity affected by the present invention;
Figure 2 is a further schematic perspective view of the apparatus according to the present invention, shown during the operation thereof;
Figure 3 is a partially cross-sectioned side view, of the cannula included in the apparatus according to the invention;
Figure 4 is a cross-sectioned view of the tubular body of said apparatus;
Figure 5 is a cross-sectional view of the tubular body of the apparatus according to a further aspect of the present invention;
Figure 6 is a side perspective view, on an enlarged scale, of the contoured tip portion of the cannula of the apparatus according to the invention;
Figure 7 is a side view, on an enlarged scale, showing the ball element of the apparatus;
Figure 8 is a side view, on an enlarged scale, showing the ball element of the apparatus, according to another aspect of the invention;
Figure 9 is a side view, on an enlarged scale, showing the ball element of the apparatus according to yet another aspect of the invention; and
Figure 10 is a partially cross-sectioned perspective view illustrating an attachment system for clamping the ball element to the tubular body.

### DESCRIPTION OF THE PREFERRED EMBODIMENTS

With reference to the number references of the above mentioned figures, the apparatus according to the invention, which has been generally indicated by the reference number 1, comprises an outer mechanical circuit, including a cannula 2, a biopump 3 and an aortic cannula 4.

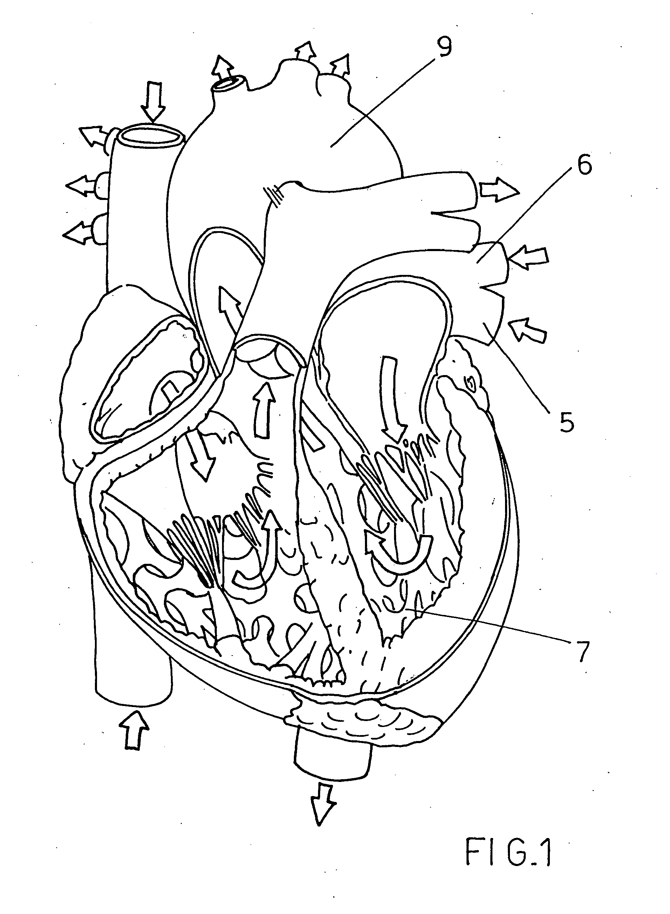

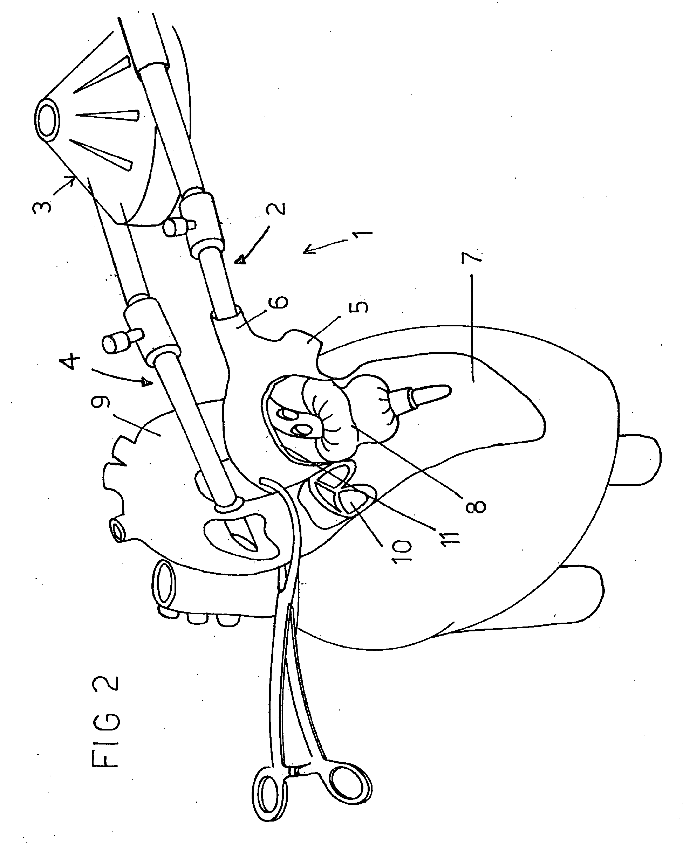

The physiologic circulatory activity herein considered provides that, as is clearly shown in figures 1 and 2, oxygened blood is conveyed, through the pulmonary veins 5 and 6, to the left atrium, and hence to the left ventricle 7.

Then, through the mitral valve 8, the blood, from the left ventricle 7, is supplied to the aorta 9, through the aortal or aortic valve 10.

This pulsating circulatory activity occurs because of the pressure gradients transmitted by the contracting heart to its chambers, and the description of which would not be important for the invention.

More specifically, the apparatus according to the invention provides to bypass a portion of the disclosed circuit, to allow to operate on the aortic valve 10 in a so-called "open sky" condition.

This is achieved by causing the blood flow to be stopped at the mitral valve and by performing a simultaneous aortic clamping, thereby directly conveying the oxygened blood to the ascending aorta through an outer mechanical circuit.

Thus, by using this technique or method, it is possible to perform a cross aortotomy to expose the aortic valve.

Actually, the cannula 2 according to the invention has been specifically designed for occluding the mitral valve 8, i.e. for preventing oxygened blood from being conveyed to the left ventricle 7, while simultaneously draining oxygened blood, present in the left atrium 11, toward the biopump 3.

The latter, which is coupled to the second infusion cannula 4, is adapted to supply the blood to the ascending aorta 9.

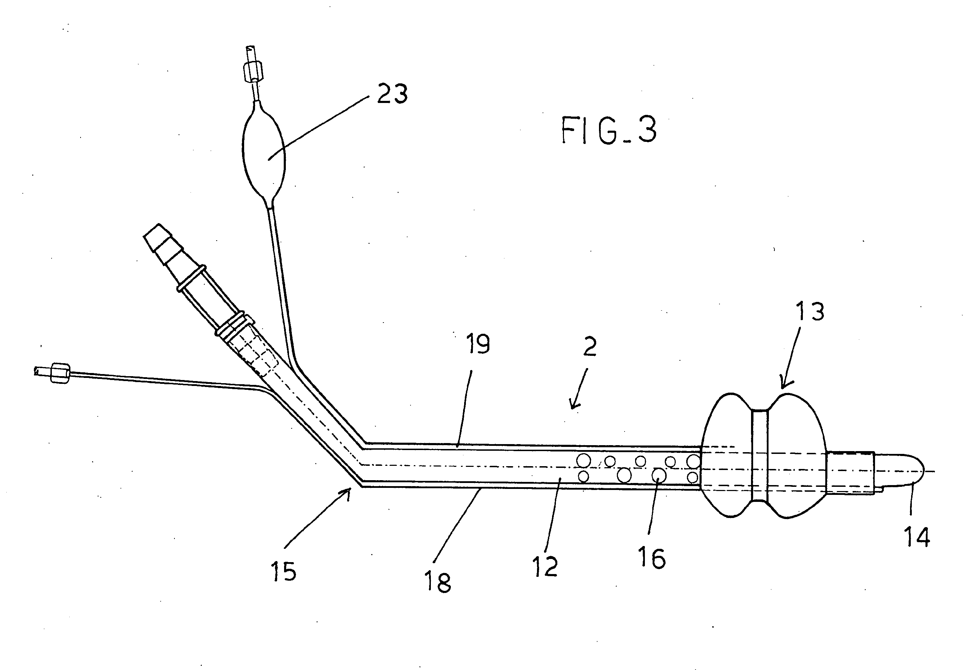

As shown, the cannula 2 comprises a tubular body 12 closed at the tip portion 14 thereof, by an occluding ball element 13, arranged near the tip portion 14, as is clearly shown in figure 3.

In this connection it should be pointed out that the tubular body can be made of polyvinylchloride or polyurethane material.

Since the cannula 2 must have a high kinking strength, i.e. it must not be occluded if it would be bent during the intervention, then the tubular body can be designed as a reinforced body, i.e. including a metal coil, having a suitable stiffness.

Satisfactory tests have been performed by using a PVC body having a 93 Sh.A hardness (for 1 mm of wall).

To facilitate the introducing operations, the cannula body can have an angled pattern from 15° to 150°, of a straight type, upstream of draining holes 16.

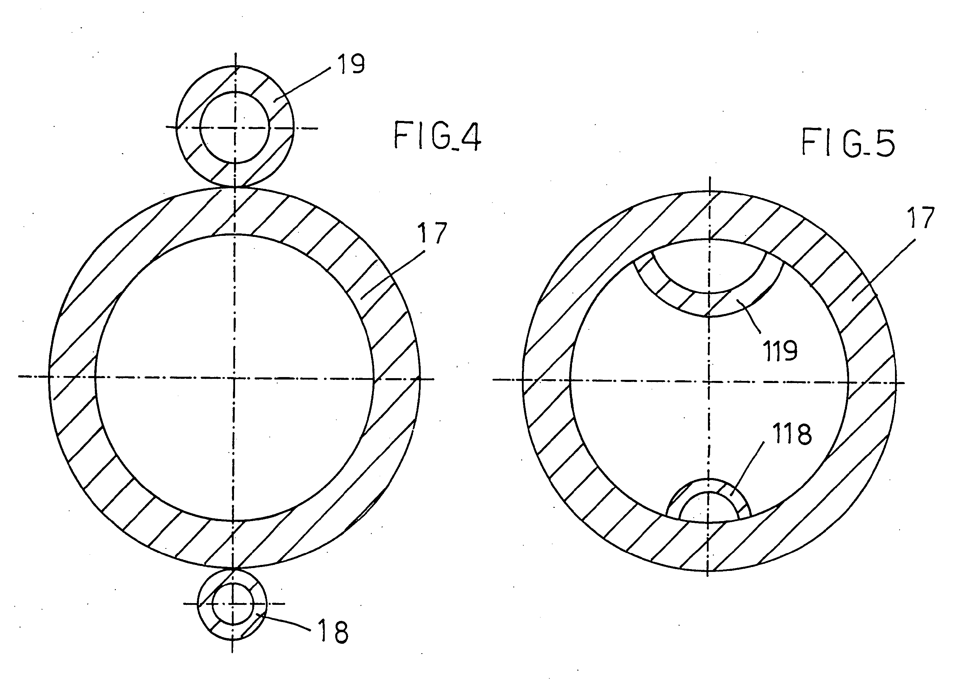

More specifically, said tubular body has a three-lumen cross-section, as is clearly shown in figures 4 and 5, said lumina being both of an outer or of an inner type.

The larger lumen, generally indicated by the reference number 17, is used for draining oxygened blood.

A first smaller lumen, either of an outer type as indicated by 18 or of an inner type as indicated by 118, is opened on the tip portion of the catheter and constitutes a means for detecting pressure in the left ventricle.

A second smaller lumen, either of an outer type as indicated by 19 or an inner type as indicated by 119, is coupled to the ball element and is used for inflating and deflating said ball elements.

On the cannula body, immediately upstream of the ball element, are provided a plurality of draining holes 16 allowing blood to be drained, through the larger lumen, to the biopump 3.

The draining holes, in particular, will expose a portion as great as possible of the draining lumen with minimum load losses.

The cannula tip portion is of a closed type, with the exception of the port toward the pressure taking lumen.

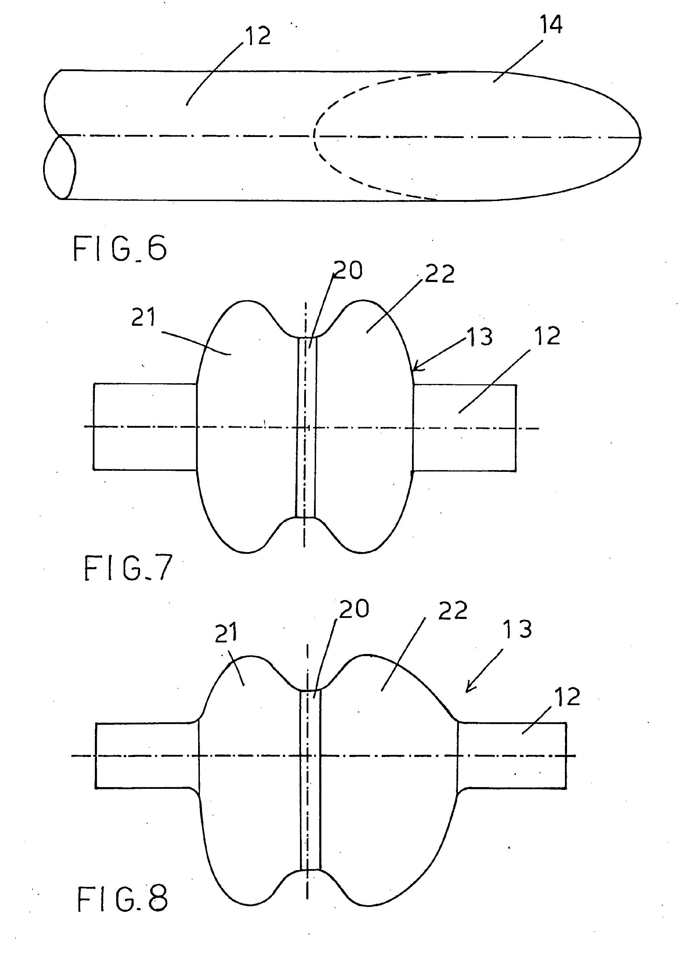

Said tip portion is so contoured as to facilitate the introduction of the cannula into the lung vein as is clearly shown in figure 6.

The closure of said tip portion is made by suitably thermoforming the cannula body.

The tip portion can constitute moreover a radio-opaque reference point, for example an inner gold ring element.

The cannula body must have a size as small as possible, for example it can have a diameter of 7.5 mm and a length of 250 mm, and being coupled to the under-pump tube by a standard fitting.

The occluding ball element 13 is so contoured as to be able of suitably close or shut-off the mitral valve in an atraumatic manner.

More specifically, it comprises two side bosses 21 and 22 having a diameter larger than that of the valve, for example 35 mm, and a recess 20 bridging said bosses and having a diameter similar to that of the valve, for example 25 mm.

The size of the ball element is designed as small as possible, in order not to prevent the blood flow in the left atrium, for example a size of 30 mm.

An exemplary embodiment is shown in figure 7.

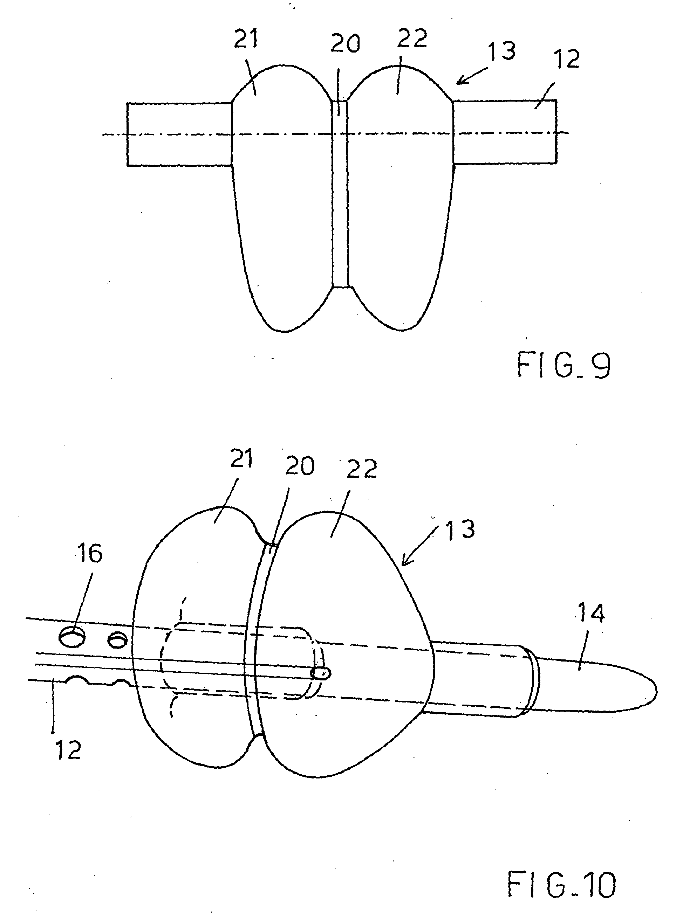

In particular, the ball element can have a symmetric contour with respect to the axis defined by the cannula, or it can also be asymmetrical, as the pattern generally shown in figure 9.

This latter pattern, which is a preferred one, is designed for preventing the ball element from hindering the valve seaming operations.

For constructional technical reasons, that portion of the ball element which is introduced into the left ventricle can have a size larger than the other portion, as shown in figure 8.

The ball element is coupled to the cannula by glueing its two open end portions.

In order to reduce the occupied space of the glued region, with respect to the hole region, the upstream end portion of the ball element is preferably glued inward of the ball element itself, as is clearly shown in figure 10.

More specifically, the ball element is glued to the cannula body as it is introduced.

To provide a low insertion profile, the cannula body, at the ball region, is preferably of concave configuration.

It should be apparent that the occluding ball must be held in an inflated condition through the overall procedure.

For achieving this, a pilot ball element 23 is preferably arranged upstream of the ball inflating line.

The occluding ball 13 must be inflated and deflated in a very easy and quick manner.

For achieving this objective, the lumen 19 of the inflating line is suitably sized, for example with a diameter of substantially 1.5 mm.

The ball must have an outer surface as soft and smooth as possible, in order not to damage the tissues, while providing a suitably strength for the introducing operations.

Moreover, the ball element 13 must be able of being patterned about the valve, without any excessive deformation.

The materials meeting the above mentioned requirements are elastomeric silicone or polyurethane.

The ball element 13 can be made by dipping molding methods.

The wall thickness must be so designed as to resist against the operation pressures; in particular, it can be, for example, of substantially 0.25 mm.

It has been found that the invention fully achieves the intended aim and objects.

In fact, the invention has provided an apparatus allowing to perform an aortic valve surgery intervention on a beating heart.

The inventive apparatus, in particular, allows to extend the advantages of a beating heart operation also to aortic valve and ascending aorta replacement and repairing interventions.

In practicing the invention, the used materials, as well as the contingent size and shapes, can be any, according to requirements and the status of the art.

## Claims

1. An apparatus for performing an aortal valvular surgery intervention on a beating heart, comprising an mechanical outer circuit, including a biopump and an infusion aortic cannula, **characterized in that** said apparatus further comprises a left atrial cannula, designed for occluding the mitral valve so as to convey oxygened blood directly to the ascending aorta through said mechanical outer circuit, thereby allowing perform a cross aortotomy operation to expose the aortic valve and to operate on said aortic valve in an open sky mode of operation.

2. An apparatus according to claim 1, **characterized in that** said atrial cannula is designed for occluding the mitral valve, i.e. preventing oxygened blood from being conveyed to the left ventricle, while draining oxygened blood, present in the left atrium, toward said biopump.

3. An apparatus according to claim 1, **characterized in that** said biopump is coupled to a second infusion cannula for conveying blood to the ascending aorta.

4. An apparatus according to claim 1, **characterized in that** said atrial cannula comprises a tubular body having a closed tip portion and provided with an occluding ball element arranged at said tip portion.

5. An apparatus according to claim 4, **characterized in that** said tubular body is made of a polyvinyl chloride or polyurethane material.

6. An apparatus according to claim 4, **characterized in that** said atrial cannula has a high kinking strength, i.e. it is not occluded as it is bent during a surgical intervention.

7. An apparatus according to claim 1, **characterized in that** said atrial cannula has an atrial cannula body of a reinforced type, which is reinforced by a metal coil.

8. An apparatus according to claim 7, **characterized in that** said atrial cannula body is made of a PVC material having a 93 Sh.A hardness (for a wall of 1 mm).

9. An apparatus according to claim 4, **characterized in that** said atrial cannula comprises a plurality of draining holes.

10. An apparatus according to claim 1, **characterized in that**, for facilitating introducing operations, said cannula body has an anguled pattern of substantially 150°, upstream of draining holes thereof.

11. An apparatus according to claim 1, **characterized in that** said tubular body has a three-lumen cross-section.

12. An apparatus according to claim 11, **characterized in that** said tubular body comprises a larger lumen and two smaller lumina.

13. An apparatus according to claim 11, **characterized in that** said lumina are either outer or inner lumina with respect to a larger lumen; said larger lumen being used for draining oxygened blood; a first smaller lumen being opened at a catheter tip portion and providing a means for detecting pressure in the left ventricle.

14. An apparatus according to claim 12, **characterized in that** a second smaller lumen is coupled to the ball element and is used for inflating and deflating said ball element.

15. An apparatus according to claim 1, **characterized in that** said cannula body comprises a plurality of draining holes arranged immediately upstream of said ball element and allowing blood to being drained, through a larger lumen, to said biopump.

16. An apparatus according to claim 15, **characterized in that** said draining holes expose a portion as great as possible of said draining lumen, thereby providing a minimum load loss.

17. An apparatus according to claim 1, **characterized in that** said cannula has a closed tip portion with the exclusion of a port toward a pressure lumen.

18. An apparatus according to claim 17, **characterized in that** said tip portion is so contoured as to facilitate an introduction of said cannula into a lung vein.

19. An apparatus according to claim 17, **characterized in that** said tip portion is closed by thermoforming said cannula body.

20. An apparatus according to claim 17, **characterized in that** said tip portion constitutes a radio-opaque reference point, for example with a gold ring in the inside thereof.

21. An apparatus according to claim 1, **characterized in that** said cannula has a cannula body with a small size, having for example a diameter of 7.5 mm and a length of 250 mm and being coupled by a fitting to an under-pump tube.

22. An apparatus according to claim 1, **characterized in that** said occluding ball element is so contoured as to shut off the mitral valve in an atraumatic manner.

23. An apparatus according to claim 1, **characterized in that** said ball element comprises two side bosses having a diameter larger than that of the valve, for example of 35 mm, and a recess bridging said two bosses and having a diameter similar to that of said valve, for example of 25 mm.

24. An apparatus according to claim 1, **characterized in that** said ball element, in order not to limit the blood flow to the left atrium, has a size of substantially 30 mm.

25. An apparatus according to claim 1, **characterized in that** said ball element is symmetrically contoured with respect to an axis defined by said cannula, or it is asymmetrical, to prevent said ball element for hindering the valve seaming operations.

26. An apparatus according to claim 1, **characterized in that that** portion of said ball element which is provided for introduction in the left ventricle has a size larger than the other portion of said ball element.

27. An apparatus according to claim 1, **characterized in that** said ball element is coupled to said cannula by glueing its two open end portions.

28. An apparatus according to claim 1, **characterized in that**, in order to reduce the size of the glueing region with respect to the hole region, the upstream end portion of said ball element is glued inward of said ball element.

29. An apparatus according to claim 1, **characterized in that** said ball element is glued to said cannula body during the introducing operation.

30. An apparatus according to claim 1, **characterized in that**, to provide a low insertion profile, said cannula body has a concave portion at the ball element region.

31. An apparatus according to claim 1, **characterized in that** said apparatus further comprises a pilot ball element upstream of the occluding ball element inflating line.

32. An apparatus according to claim 31, **characterized in that** said inflating line has a lumen which is so designed as to allow said occluding ball element to being inflated and deflated in an easy and quick manner.

33. An apparatus according to claim 32, **characterized in that** said inflating line lumen has a diameter of substantially 1.5 mm.

34. An apparatus according to claim 31, **characterized in that** said ball element has a soft and smooth outer surface in order not to damage the tissues and to resist against the introducing operations.

35. An apparatus according to claim 31, **characterized in that** said ball element is designed for being patterned about said valve without excessively deforming.

36. An apparatus according to claim 31, **characterized in that** said ball element is made of an elastomeric silicone or polyurethane material.

37. An apparatus according to claim 31, **characterized in that** said ball element is made by a dipping molding method.

38. An apparatus according to claim 31, **characterized in that** said ball element has a wall having a thickness adapted to resist against the operating pressure, said thickness being substantially of 0.25 mm.

39. An apparatus according to claim 1, **characterized in that** said apparatus comprises a straight type of atrial cannula.
